# EUROPEAN PATENT SPECIFICATION

(11) **EP 2 307 065 B1**
(45) Date of publication and mention of the grant of the patent: **14.02.2018**
(21) Application number: 09774553.3
(22) Date of filing: 02.07.2009
(51) Int. Cl.: A61L 27/18, A61L 27/38, A61L 27/58, A61L 26/00, A61L 27/20, A61L 27/24, A61L 27/26, A61L 27/50

(54) **COMPOSITIONS AND METHODS FOR TISSUE FILLING AND REGENERATION**
ZUSAMMENSETZUNGEN UND VERFAHREN FÜR GEWEBEAUFFÜLLUNG UND GEWEBEREGENERATION
COMPOSITIONS ET PROCÉDÉS DE REMPLISSAGE ET DE RÉGÉNÉRATION TISSULAIRES

(30) Priority: 02.07.2008 US 77683 P
(43) Date of publication of application: 13.04.2011
(60) Divisional application: 13184537.2
(73) Proprietor: ALLERGAN, INC., Irvine, CA 92612 (US)
(72) Inventor: VAN EPPS, Dennis, E., Goleta CA 93117 (US)
(74) Representative: Hoffmann Eitle
(86) International application number: PCT/US2009/049597
(87) International publication number: WO 2010/003104

(56) References cited:
- WO-A1-99/07416
- WO-A1-2008/148026
- US-A1- 2007 104 692
- CHO S-W ET AL: "Engineering of volume-stable adipose tissues" BIOMATERIALS, ELSEVIER SCIENCE PUBLISHERS BV., BARKING, GB LNKD- DOI:10.1016/J.BIOMATERIALS.2004.09.013, vol. 26, no. 17, 1 June 2005 (2005-06-01), pages 3577-3585, XP025280561 ISSN: 0142-9612 [retrieved on 2005-06-01]
- CHOI Y S ET AL: "Adipose tissue engineering using mesenchymal stem cells attached to injectable PLGA spheres" BIOMATERIALS, ELSEVIER SCIENCE PUBLISHERS BV., BARKING, GB LNKD- DOI:10.1016/J.BIOMATERIALS.2005.02.022, vol. 26, no. 29, 1 October 2005 (2005-10-01), pages 5855-5863, XP025280137 ISSN: 0142-9612 [retrieved on 2005-10-01]
- HEMMRICH ET AL: "Autologous In Vivo Adipose Tissue Engineering in Hyaluronan-Based Gels-A Pilot Study" JOURNAL OF SURGICAL RESEARCH, ACADEMIC PRESS INC., SAN DIEGO, CA, US LNKD- DOI:10.1016/J.JSS.2007.03.017, vol. 144, no. 1, 11 December 2007 (2007-12-11), pages 82-88, XP022385921 ISSN: 0022-4804
- HEIMBURG VON D ET AL: "INFLUENCE OF DIFFERENT BIODEGRADABLE CARRIERS ON THE IN VIVO BEHAVIOR OF HUMAN ADIPOSE PRECURSOR CELLS" PLASTIC AND RECONSTRUCTIVE SURGERY, WILLIAMS AND WILKINS CO., BALTIMORE, MD, US LNKD- DOI:10.1097/00006534-200108000-00020, vol. 108, no. 2, 1 August 2001 (2001-08-01), pages 411-422, XP009032694 ISSN: 0032-1052
- HEMMRICH K ET AL: "Implantation of preadipocyte-loaded hyaluronic acid-based scaffolds into nude mice to evaluate potential for soft tissue engineering", BIOMATERIALS, ELSEVIER SCIENCE PUBLISHERS BV., BARKING, GB, vol. 26, no. 34, 1 December 2005 (2005-12-01), pages 7025-7037, XP027767674, ISSN: 0142-9612 [retrieved on 2005-12-01]
- PARK S ET AL: "Biological characterization of EDC-crosslinked collagen-hyaluronic acid matrix in dermal tissue restoration", BIOMATERIALS, ELSEVIER SCIENCE PUBLISHERS BV., BARKING, GB, vol. 24, no. 9, 1 April 2003 (2003-04-01), pages 1631-1641, XP004404219, ISSN: 0142-9612, DOI: 10.1016/S0142-9612(02)00550-1
- PARK S-N ET AL: "Characterization of porous collagen/hyaluronic acid scaffold modified by 1-ethyl-3-(3-dimethylaminopropyl)carbodiim ide cross-linking", BIOMATERIALS, ELSEVIER SCIENCE PUBLISHERS BV., BARKING, GB, vol. 23, no. 4, 15 February 2002 (2002-02-15), pages 1205-1212, XP004348139, ISSN: 0142-9612, DOI: 10.1016/S0142-9612(01)00235-6

## Description

### Background

The present invention generally relates to tissue filling, augmentation and regeneration, and more specifically provides a combined cell and filler composition for use in a method of therapeutically augmenting soft tissue in a mammal.

Various products have been injected into the human body to augment soft tissue and correct skin defects. Early examples of such products include paraffin, petrolatum, vegetable oils, lanolin, bees wax, and silicone.

Bovine and human collagen have gained widespread use as injectable materials for soft tissue augmentation and filling. Collagen, the principal extracellular structural protein of the animal body, has been used as an implant material to replace or augment connective tissue, such as skin, tendon, cartilage and bone. Additionally, collagen has been injected or implanted into the human body for cosmetic purposes for a number of years.

Hyaluronic acid (HA) is a glycosaminoglycan that is naturally found in the human body and is widely distributed throughout connective, epithelial, and neural tissues. In 2003, the Food and Drug Administration approved HA-based injections for correcting facial wrinkles.

Schroeder et al., U.S. Patent Application Serial No. No. 12/247,175, filed October 7, 2008 (published as US 2009/0093755 A1), describes crosslinked hyaluronic acid and collagen, for augmenting soft tissue in mammals and methods for preparing the same.

US 2007/0104692 A1 discloses a method of treating a breast defect comprising differentiating an isolated human adipose-derived stromal cell into a breast tissue progenitor cell, and administering the progenitor cell to the defective area of the breast.

HEMMRICH ET AL disclose injectable compositions comprising amidated hyaluronan gels mixed with stem-cell derived precursors of adipose tissue for use in soft tissue reconstruction including correction of contour incongruences (Autologous In Vivo Adipose Tissue Engineering in Hyaluronan-Based Gels-A Pilot Study",JOURNAL OF SURGICAL RESEARCH, ACADEMIC PRESS INC., SAN DIEGO, CA, US, vol. 144, no. 1, 11 December 2007 (2007-12-11), pages 82-88).

### Summary of the Invention

The present invention generally provides improved compositions for augmenting and repairing the skin and soft tissues for therapeutic purposes. The present compositions are useful for filling and regeneration of soft tissues in a mammalian patient, for example a human being.

In a broad aspect, the invention provides an injectable composition for use in a method of therapeutically augmenting soft tissue in a mammal by injecting the composition into a target region of the mammal where tissue volumizing and tissue regeneration is desirable, the target region being adjacent to a prosthetic implant;
wherein the injectable composition comprises
a cellular component including living cells; and
a degradable filler component conducive to maintaining viability of the cells, the degradable filler component comprising crosslinked hyaluronic acid.

The composition is capable of providing both immediate tissue filling and long term tissue regeneration.

In accordance with one embodiment of the invention, the cellular component of the present compositions comprises cells selected from the group consisting of stem cell in-situ culture, progenitor cells, adipose cells, adipose-derived stem cells, mesenchemal stem cells, endothelial cell precursors, proliferating cells, differentiation-committed cells, and regenerative cells.

The injectable filling and regenerating composition may be prepared by a method including the step of combining, for example, mixing, human stem cells with a hyaluronic acid-based filler material or premixing the cellular component with a soluble form of the material prior to gelation. Addition of integrin binding components provide adhesion and signals to retain viability or enhance cell growth. Such components include one or more of laminin, vitronectin, fibronectin, elastin and peptides and analogs thereof.

The composition may be contained in a syringe of a kit which also comprises a cannula and instructions for use.

The composition is useful as a component of a kit together with a tool for extracting autologous cells from a patient and an injector device for enabling injection of a mixture of the composition and extracted autologous cells into a target region of the patient.

### Brief Description of the Drawings

Fig. 1 is a schematic view of a kit.
Fig. 2 is a schematic view of another kit.

### Detailed Description

The present invention provides a highly useful, safe and effective composition for soft tissue augmentation in a mammal, for example, a human being. The injectable composition is useful for substantially immediate tissue filling or volumizing of tissue as well as support for long term tissue regeneration.

The composition of the present invention can be used for breast augmentation and reconstruction where a combination of volume and tissue regeneration may be desired. Similarly, filling of any tissue void either natural or created by surgical procedure for removal of tissue, corticosteroid treatment, immunologic reaction resulting in lipoatrophy, tissue damage resulting from impact injuries, radio or chemical or drug treatment where there is a desire to both fill and regenerate tissue at a particular site. Similarly, the present composition can be used as a means for reducing scar tissue as a result of the active vascularization and dissolution of scar tissue, either as a preventative or post treatment procedure based on the delivery of viable fat-derived cells or stem or progenitor cells to the site.

The filler component effectively provides volume and support for viability and growth of the cells when the composition is injected into a target region of a patient.

The present composition, when injected into a target region of a patient, provides relatively long term, for example, greater than six months, of increased tissue filling and volumizing, relative to a substantially identical composition which does not include a cellular component. The present composition also provides the support, structure and space within the tissue to allow for growth and regeneration of tissue. The cellular component in the present composition also provides for the production or stimulation of cytokines and intrinsic stimulators of tissue growth and maintenance at the site of injection.

The cellular component of the present composition preferably comprises adipose-derived progenitor cells, for example, adipose-derived stem cells. In some embodiments of the invention, the composition includes autologous cells, for example, autologous adipose-derived adult stem and/or progenitor cells. The cellular component provides relatively long-term tissue regeneration when combined with the filler component after the composition has been injected into a patient.

The filler component of the present composition comprises crosslinked hyaluronic acid that can provide substantially immediate or short term tissue filling and an environment conducive to cell or tissue growth.

The crosslinked hyaluronic acid is able to absorb water and expand once injected into the body, to provide space for cell growth to enhance tissue regeneration.

In one embodiment, the filler component may further include one or more additional materials or agents which are components of a natural extracellular matrix or peptides, derivatives or analogs of integrin binding molecules, that are capable of optimizing implanted cell viability and/or sustaining cell growth for a relatively long term or sustained period of time after the injection of the composition.

The filler component may comprise crosslinked hyaluronic acid combined with one or more other beneficial materials, for example, integrin binding molecules, integrin binding derivatives or analogs thereof, or peptides or peptide analogs with the potential to bind to integrins on the injected cell population. Such materials may be selected based on their ability to bind to growth factor receptors to thereby stimulate cell growth, for example, the injected adipose cells and/or the influx of intrinsic tissue progenitors or cytokines.

Suitable integrin binding proteins useful in the context of the present invention include, but are not limited to, collagen, elastin, laminin, vimentin, and non-protein cell binding components such as heparin sulfate or other materials.

In one embodiment of the invention, the filler component comprises a crosslinked hyaluronic acid (HA)-based composition, or a crosslinked HA and collagen based composition, such as described in Schroeder et al., U.S. Patent Application No. 12/247,175, filed on October 7, 2008, having common assignee herewith.

In some embodiments, the filler component further comprises a thermo-sensitive polymer, for example, a sol-gel transforming hydrogel, for example, a sol-gel transforming polysaccharide hydrogel composition. Suitable sol-gel transforming hydrogels exhibit the properties of being in the state of a liquid (sol) at room temperature or below, but form a hydrogel which is sufficiently gel-like to hold its shape, when at physiological temperature, or body temperature (37°C). Suitable sol-gel transforming materials include the natural biopolymer chitosan and derivatives thereof. In combination with glycerol phosphate (GP-sodium salt), this cationic polyelectrolyte becomes thermosensitive in diluted acids and can undergo gelation around body temperature.

Exemplary thermogelling hydrophobic blocks useful in the filler components in accordance with the invention include, for example, but are not limited to poly(propylene oxide), poly(lactide-co-glycolic acid), poly(N-isopropylacrylamide), poly(propylene fumarate), poly(caprolactone), poly(urethane) and poly(organophosphazene).

The compositions of the present invention are useful for tissue "sculpting", tissue replacement or regeneration, improving scar formation or reducing existing scar tissue or increasing tissue elasticity tissue regeneration after mastectomy or lumpectomy, and other clinical settings where volumizing and tissue regeneration are desirable. In some specific embodiments of the invention, the compositions are useful for treatment of damaged tissue, for example, radiation-induced tissue damage, steroid induced lipoatrophy or surgical or trauma induced soft tissue loss. For example, the compositions can be used as an effective means of delivering cells to an area of radiation-induced tissue damage in order to reduce scarring, replenish lost tissue progenitors, or generally improve overall patient outcome. The present compositions can further be used in the treatment of existing scar tissue, or for wound covering and prevention of scar formation. Some of the present compositions are useful in the treatment of radiation-induced tissue damage.

The composition of the invention is for use in a method of filling tissue located at or near a previously implanted prosthetic implant, for example, a conventional breast implant. The method may include the step of placing a prosthetic implant in a patient and subsequently placing a composition comprising a combined sol-gel transforming component and cellular component adjacent the breast implant to "sculpt" or smooth the breast, for example, to reduce or eliminate depressions or other anomalies in the breast tissue near the implant.

The composition may be prepared by providing a filler material,providing a cellular material and combining or mixing the filler material with the cellular material to produce a useful filling and regenerating composition.

For example, the step of providing the filler component includes producing a HA/collagen filler component. For example, the step includes contacting HA with a cross-linker to allow cross-linking of the HA by the cross-linker, thereby forming a first composition, contacting the first composition with collagen to allow cross-linking of the collagen by the cross-linker, thereby forming a second composition, and contacting the second composition with a HA solution to allow cross-linking of the HA in the solution by the cross-linker, thereby producing an HA/collagen filler component. The next step includes combining or mixing the HA/collagen filler component with a cellular component including a preparation of living cells, thereby forming an injectable filling and regenerating composition.

In one especially advantageous method, the step of providing a cellular component may include obtaining living cells, preferably including adult stem cells, to be mixed with the filler component. For example, the invention may include the steps of extracting tissue, for example, adipose tissue, from a patient to be treated with the filling and regenerating composition. The extracted tissue may be processed in a manner suitable for obtaining, for example, substantially isolating, adult stem cells therefrom.

In one embodiment of the invention, adipose derived stem cells form at least a portion of the cellular component of the injectable filling and regenerating composition. Adipose cells may be harvested from a patient by conventional liposuction or aspiration techniques. The removed fat tissue would be separated, for example, by centrifugation, to yield a tissue fluid layer, a fat cell layer and a layer of oils or lipids. The fat cell layer is harvested.

Centrifugation may be accomplished in a syringe, a bag like a "blood bag", or an automated centrifugation system such as available from Cytori Therapeutics.

Any suitable, conventional mechanism which can provide separation of cells from extracted tissue may be utilized. For example, simple gravity sedimentation techniques may be used to provide separation of cells from unwanted or unneeded acellular components. It is further contemplated that purified or enriched cell products may also be used such as enriched mesenchymal stem cells or other stem or progenitor cell types capable of differentiation to mature tissue cells.

It is advantageous to utilize cellular materials that are not cultured for any significant period of time. For example, cellular material which is extracted from a patient can be used as a component of a filler product for the patient within the same day. In other methods, cellular material is cryopreserved and thawed for subsequent use as a component of a filler product for injection at a later time. Cultured cells may be utilized by incubation in a nutrient media with or without growth factors that would support the viability and expansion of progenitor cells capable of forming fat, blood vessel cells, dermal cells or muscle cells. Processing is described above and generally cells would be utilized in a relatively short period of time that day.

More specifically, in some methods, cell material is stored or preserved, for example, using cryopreservation, prior to mixing with the filler component. Cryopreservation involves controlled freezing and storage of cellular material in liquid nitrogen or in freezers capable of reaching temperatures of -80°C or lower.

Prior to being mixed with the filler/volumizing component, the cryopreserved cellular material is rapidly thawed, for example, at a temperature of about 37°C. In some methods, centrifugation of the thawed cellular material may be needed to remove any cellular debris, lipid or acellular material prior to mixing the thawed cryopreserved cells with the filler/volumizer component.

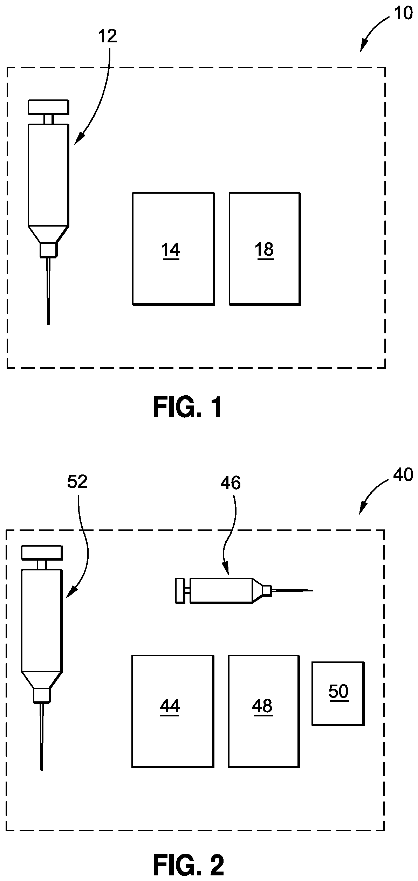

Turning now to Fig. 1, a kit 10 for use in filling and regenerating tissue is provided. The kit 10 may comprise an injection device, for example, a syringe 12, an injectable composition 14 which is a cell/filler composition for tissue filling and regeneration as described elsewhere herein, and instructions for use 18. For example, composition 14 comprises a cellular component including living progenitor cells and the filler component comprises hyaluronic acid-based gel.

Turning now to Fig. 2, a kit 40 is provided which comprises a filler component 44, a suitable tool 46 for extracting cells from a region of a body of a patient, and instructions for use 48. The kit 40 may further comprise a mixing vessel 50 for combining cells extracted with the tool 46 with the filler component 44 to produce an injectable composition which is a cell/filler mixture as described elsewhere herein. The kit 40 may further include an injection device, for example a syringe 52, for enabling injection of the cell/filler mixture into the patient, for example, in a different region, for example a breast region, of the body of the patient, wherein the different region would be benefited from both fill volume and tissue regeneration.

In some embodiments of the invention, the composition comprises a cellular component and a gel component, wherein the cellular component comprises cells mixed into or homogenized with the gel component. Advantageous uses of this embodiment include coating a wound or existing scar tissue to accelerate healing, prevent or reduce occurrence of scarring, or possibly reverse photo-, thermal- or radiation-induced tissue damage. Such a composition could be injected around and/or under an existing scar to provide for regeneration of tissue and softening of the scarred region.

Applications of the present compositions include their use in sculpting, tissue replacement or regeneration, improving scar formation, tissue regeneration after mastectomy or lumpectomy, and other clinical settings where volume and tissue regeneration are desirable. The present compositions are used in conjunction with a prosthetic implant, for example a conventional breast implant, as a tissue filler, for example, to smooth or fill depressions and/or other areas of the body that may occur adjacent the prosthetic implant.

### Reference Example

A composition useful for filling and regenerating soft tissue is prepared as follows:
Adipose tissue is collected using typical liposuction procedures. Lipoaspirate may be further separated by centrifugation to remove excess fluid or free lipid. Cellular material containing fat cells, endoothelial cells, mesymchymal cells and stem and progenitor cells would be collected and adjusted if necessary with a compatible solution to an appropriate cell concentration suitable for mixing with the filler component. Mixing could either be by agitation of the two components or by utilization of a bifurcated or dual chamber system that would mix the two components prior to or during the injection procedure. If a sol-gel transformation is involved with the filler component, a catalyst such as a salt or pH change would be achieved when the two components are mixed. If a thermal dependent sol-gel transformation is required for the filler, this may be achieved by an in vivo temperature shift post injection or by external manipulation of the temperature of the mixture prior to injection. Injection into the target site may be achieved using a needle or cannula attached to a syringe or delivery device.

## Claims

1. An injectable composition for use in a method of therapeutically augmenting soft tissue in a mammal by injecting the composition into a target region of the mammal where tissue volumizing and tissue regeneration is desirable, the target region being adjacent to a prosthetic implant;
wherein the injectable composition comprises
a cellular component including living cells; and
a degradable filler component conducive to maintaining viability of the cells, the degradable filler component comprising crosslinked hyaluronic acid.

2. A composition for use according to Claim 1, wherein the cellular component comprises cells selected from the group consisting of stem cell in-situ culture, progenitor cells, adipose-derived stem cells, endothelial cell precursors, proliferating cells, differentiation-committed cells, and regenerative cells.

## Patentansprüche

1. Injizierbare Zusammensetzung zur Verwendung in einem Verfahren des therapeutischen Verstärkens von Weichgewebe in einem Säuger durch Injizieren der Zusammensetzung in eine Zielregion des Säugers, in der Gewebe volumenvergrößert wird und Geweberegeneration erwünscht ist, wobei die Zielregion an ein prothetisches Implantat angrenzt;
wobei die injizierbare Zusammensetzung
eine zelluläre Komponente, die lebende Zellen enthält; und
eine abbaubare Füllstoffkomponente, die geeignet ist um die Lebensfähigkeit der Zellen aufrechtzuerhalten, umfasst, wobei die abbaubare Füllstoffkomponente vernetzte Hyaluronsäure umfasst.

2. Zusammensetzung zur Verwendung gemäß Anspruch 1, wobei die zelluläre Komponente Zellen, ausgewählt aus der Gruppe bestehend aus Stammzellen-in-situ-Kultur, Vorläuferzellen, aus Fettgewebe stammenden Stammzellen, Endothelzellvorläufern, proliferierenden Zellen, differenzierungsbestimmten Zellen und regenerative Zellen, umfasst.

## Revendications

1. Composition injectable pour une utilisation dans un procédé d'augmentation thérapeutique de tissus mous chez un mammifère en injectant la composition dans une région cible du mammifère où donner du volume aux tissus et régénérer les tissus est souhaitable, la région cible étant adjacente à un implant prothétique ;
dans laquelle la composition injectable comprend
un composant cellulaire comprenant des cellules vivantes ; et
un composant de charge dégradable favorisant le maintien de la viabilité des cellules, le composant de charge dégradable comprenant de l'acide hyaluronique réticulé.

2. Composition pour une utilisation selon la revendication 1, dans laquelle le composant cellulaire comprend des cellules choisies dans le groupe consistant en une culture in situ de cellules souches, des cellules progénitrices, des cellules souches dérivées d'adipose, des précurseurs de cellules endothéliales, des cellules proliférantes, des cellules engagées dans la différenciation et des cellules régénératives.
